# EUROPEAN PATENT APPLICATION

(11) **EP 2 397 463 A1**
(43) Date of publication of application: **21.12.2011**
(21) Application number: 10166224.5
(22) Date of filing: 16.06.2010
(51) Int. Cl.: C07C 273/04, B01J 10/00

(54) **Method for revamping a self-stripping urea plant**

(71) Applicant: Urea Casale SA, 6900 Lugano-Besso (CH)
(72) Inventor: Scotto, Andrea, 6932 Breganzona (CH); Gabbiadini, Serena, 20125 Milano (IT); Bertini, Paolo, 6900 Lugano (CH)
(74) Representative: Zardi, Marco

(57) **Abstract**

A method for revamping a self-stripping urea plant is disclosed, where an intermediate recovery section (30) is installed between the existing high-pressure section and medium-pressure section; a stream of urea solution (15A), which is delivered by the high pressure section and originally directed to the medium pressure section, is at least partly redirected to said intermediate section (30), and a flow of concentrated solution (15B) from said intermediate section is directed to said medium pressure section, the intermediate section operating at a pressure between those of the high- and medium-pressure sections. A related process and plant are also disclosed.

## Description

### Field of the invention

The present invention refers to a method for revamping of a self-stripping urea plant.

### Prior Art

A disclosure of the self-stripping or thermal-stripping process and related plant can be found e.g. in GB 1 542 371. The process is also referred to by the name of its developer Snamprogetti. A description is also found in Kirk-Othmer, Encyclopedia of Chemical Technology, 3rd Ed, Vol 23, p. 548-562.

A self-stripping urea plant comprises: a high-pressure loop, usually operating at about 140 - 160 bar; a medium-pressure (MP) section and a low-pressure (LP) recovery section where urea solution delivered by the high-pressure loop is treated to decompose carbamate and recycle ammonia. As an indication, the MP section may operate around 10 - 20 bar and the LP section may operate at a few (e.g. 3-4) bar pressure. Common values are for example 150 - 17 - 3.5 bar for high, medium and low-pressure section, respectively.

The high-pressure loop typically comprises a synthesis reactor, a steam-heated stripper, and a horizontal kettle condenser. All these items operate substantially at the same pressure.

Ammonia and CO₂ are reacted in said synthesis reactor obtaining a urea aqueous solution, comprising ammonia and unconverted ammonium carbamate; said solution is heated in the high-pressure stripper to decompose the carbamate and recover ammonia; a vapour phase containing ammonia and CO₂ produced in the stripper is condensed in the high-pressure condenser, and recycled to said reactor. The stripping process takes place with the help of heat furnished e.g. by hot steam, and without addition of an external stripping medium such as carbon dioxide (thermal stripping), or using part of the ammonia available in the solution, as a stripping agent (ammonia stripping).

In this description, the term self-stripping urea plant is used to mean a urea plant comprising a high-pressure thermal or ammonia stripping section.

There is a strong interest in the revamping of existing self-stripping urea plants. When an existing plant is revamped, one should try to maintain, whenever possible, the available equipments, for economical reasons. The applicant has noted that a major bottleneck is the capacity of the medium- and low-pressure sections. For example, when the high-pressure loop is boosted, a greater flow rate of urea solution is discharged to the downstream MP/LP sections, which means a considerably larger quantity of carbamate to decompose and ammonia to recover. The available components of said MP and LP sections, including decomposers, condensers, etc... are usually close to their maximum capacity and are not able to comply with this increased duty. Hence, the bottleneck is actually found in the MP and LP sections. Revamping also the MP and/or the LP section, together with the high-pressure section, would involve the addition or replacement of several pressure vessels and then would involve a considerable cost that may render the overall intervention less attractive.

### Summary of the invention

The invention is aimed to solve the above drawback and to provide an efficient solution for boosting a self-stripping urea plant. The idea underlying the invention is to add a further pressure level to the processing of the urea solution delivered by the HP section. A further step of carbamate decomposition and ammonia recovery is added upstream the MP section, i.e. at a pressure greater than the original medium pressure of the plant.

Hence is is proposed a new method for modifying a self-stripping urea plant, said plant comprising a high pressure urea synthesis section which includes at least a reactor, a thermal or ammonia stripper and a condenser, a medium pressure treatment section and a low pressure recovery section, the method being characterized by the following steps:
- an intermediate section is installed and connected between the high-pressure synthesis section and the medium-pressure section;
- a stream of urea solution, which is delivered by the high pressure section and originally directed to the medium pressure section, is at least partly redirected to said intermediate section;
- a flow of concentrated solution from said intermediate section is directed to said medium pressure section;
- said intermediate section being suitable to decompose the carbamate and recover ammonia contained in said urea solution at an intermediate pressure which is lower than working pressure of said synthesis section and greater than the working pressure of said medium pressure section, obtaining said flow of concentrated solution at said intermediate pressure.

The added intermediate section comprises preferably at least a decomposition section and an ammonia recovery section, realized e.g. with a decomposer and an ammonia condenser, respectively. The steps of redirecting the urea solution to the intermediate section, and directing the concentrated solution to the downstream MP section, are carried out by providing the necessary piping, valves, pumps and auxiliary equipments, according to a per se known technique.

In a preferred embodiment, the full flow of urea solution delivered by the high pressure section is redirected to the newly installed intermediate section.

In some embodiments, the capacity of the high-pressure synthesis section can be increased. This increase of capacity may be achieved by modifying any of the components of the synthesis section such as reactor, stripper and condenser, or by adding further equipments to the synthesis section.

The nominal pressure of said intermediate section is preferably in the range 30 to 100 bar, more preferably 70 to 80 bar. In a preferred embodiment, the equipments of the intermediate section include at least a decomposer, a condenser and a rectification column.

The advantage of the invention is that the MP and LP section are de-bottlenecked by redirecting at least a part of the urea solution coming from the high-pressure stripper, to the added intermediate section. If the synthesis section is boosted, the capacity of said synthesis section can be consistently increased while, at the same time, the duty of the downstream MP and LP sections is maintained substantially unaltered. The extra duty, in fact, is accomplished by the newly-installed intermediate section. In other words, the invention allows to boost the HP section in a significant manner and to debottleneck the downstream MP and LP section by the addition of the intermediate section.

The modified plant operates, in practice, with a modified self-stripping process with a further pressure level, compared to the conventional process. Said process is a further aspect of the invention, according to claim 7. Another aspect of the invention is a urea plant natively designed to operate according to the new process, as set out in claim 10.

Further characteristics and advantages of the invention shall become clearer from the following description of some example embodiments, with reference to the attached drawings.

### Brief description of the drawings

Fig. 1 is a scheme of a conventional self-stripping plant.
Fig. 2 is a scheme of the plant of Fig. 1 modified according to the invention.

### Detailed description of preferred embodiments

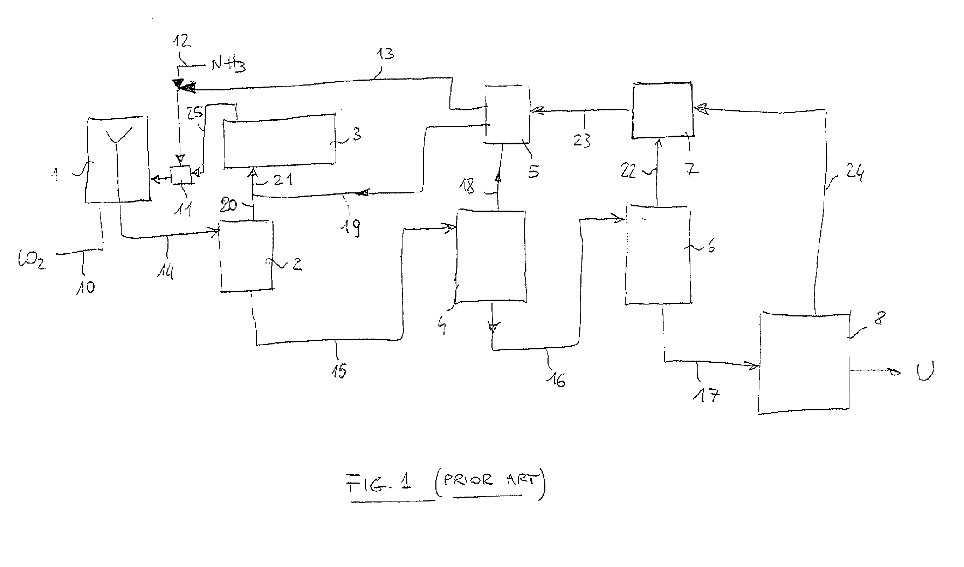

Referring to the conventional scheme of Fig. 1, the high-pressure section of the plant comprises a reactor 1, a thermal stripper or ammonia stripper 2, and a condenser 3. The medium pressure (MP) section of the plant comprises a decomposition section 4 and an ammonia recovery section, which is indicated as block 5. The low pressure (LP) section comprises a decomposition section 6 and a low pressure ammonia recovery section 7. Block 8 designates a vacuum concentration section.

A carbon dioxide feed is sent to the synthesis reactor 1 via line 10. An ejector 11 feeds the reactor 1 with fresh ammonia 12 and ammonia 13 recovered from the MP and LP sections. A urea solution 14 comprising urea, unconverted carbamate and free ammonia is discharged from reactor 1.

The urea solution 14 is sent to the stripper 2, where said solution is decomposed with the help of heat. A concentrated urea solution 15, produced in the stripper 2 and containing residual carbamate and ammonia, is fed to MP decomposition section 4; further concentrated urea solution 16 is fed to the LP decomposition section 6. The solution 17 delivered by the LP section is further treated in the vacuum section 8, obtaining the urea product U.

A vapour phase 18 separated in the MP decomposition section 4, and containing mainly CO₂, ammonia and water, is treated in the ammonia recovery section 5, which delivers a liquid ammonia stream 13 and a solution 19 containing residual carbamate. To this purpose, the section 4 may comprise for example a rectifying column where excess ammonia in the stream 18 is separated, and an ammonia condenser where the liquid ammonia stream 13 is produced. Bottom liquid from said rectifying column forms the solution 19.

Said solution 19 is mixed with overhead vapors 20 released by the stripper 2; the resulting mixed stream 21 is condensed in the high-pressure condenser 3, obtaining a condensate stream which is returned to the reactor 1 via the ejector 11.

A vapour phase 22 separated in the LP decomposing section 6 is condensed in the LP ammonia recovery section 7, obtaining ammonia 23 which is pumped to the MP recovery section 5, or eventually mixed with the vapour stream 18. Further ammonia is recycled from the vacuum section 8 to the LP section of the plant, via line 24.

The non-condensable gases in the output stream 25 of the condenser 6 are removed in a separator (not shown) which is located upstream the ejector 11.

The stripper 2 is typically a tube-bundle, steam-heated exchanger. The solution 14 from reactor 1 forms a liquid film inside the tubes of the tube bundle, whereas hot steam flowing outside the tubes supplies the heat necessary to decompose the carbamate contained in the solution. Ammonia and carbon dioxide are recovered in gaseous phase 20 at the top of the stripper. The condenser 3 is usually a horizontal shell-and-tube kettle unit, where condensation is effected on the tube side, and the condensation heat is used to produce steam.

The above details are known to a skilled person, and no further described.

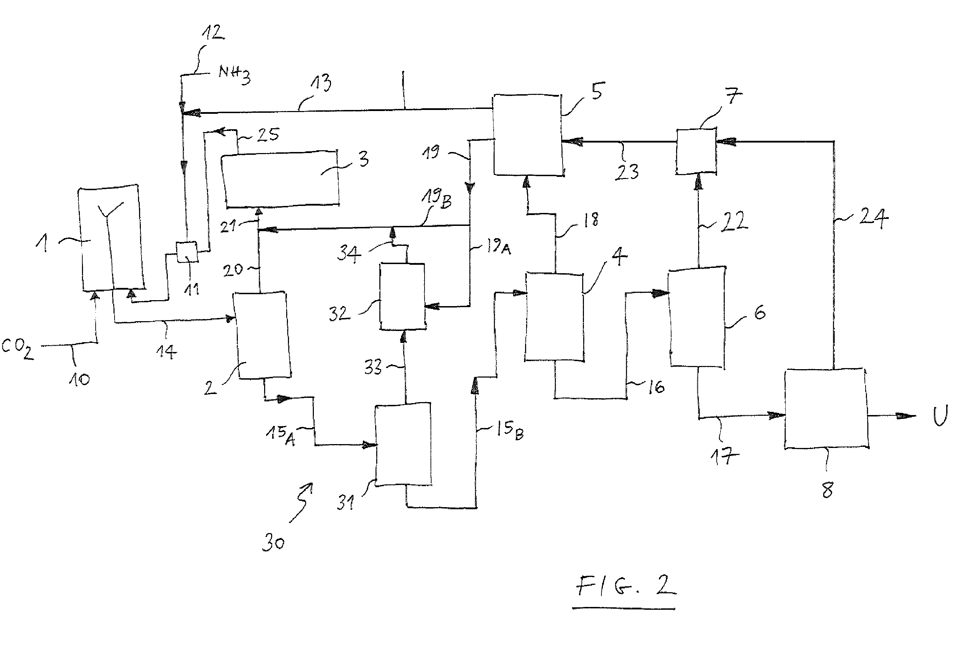

Fig. 2 discloses a scheme of the plant modified according to the invention. The urea solution from the stripper 2 is now indicated as stream 15A, which may be larger than original stream 15 in embodiments where the capacity of the high pressure synthesis section is increased.

The concentrated solution from the stripper 2, namely the urea solution 15A, which is originally directed to the MP decomposing section 4, is now redirected to a newly-installed intermediate section 30, comprising a decomposing section 31 and an ammonia recovery section 32. In Fig. 2 the full output of urea solution, coming from the boosted high pressure section 1, is redirected to said intermediate section 30, although other embodiments of the invention provide that a portion of stream 15A is redirected to the new section 30, and a remaining portion passes from stripper 2 to the MP section.

The working pressure of said intermediate section 30 is between the pressure of the high-pressure section and that of MP section, for example 30 to 100 bar and more preferably 70 to 80 bar. For example the high pressure of reactor 1, stripper 2 and condenser 3 is greater than 100 bar, the medium pressure is in the range 10 to 20 bar, the low pressure is less than 5 bar. Equipments of the sections operating at a certain pressure, such as reactor 1, stripper 2 and condenser 3, operate substantially all at the same pressure, with small differences that may be due to pressure losses in the connections, valves, etc...

The decomposition section 31 comprises at least a decomposer and operated substantially in the same way as the MP section 4, producing the concentrated solution 15B and a vapour phase 33 containing CO2, ammonia and water, which is condensed in the recovery section 32. A portion 19A of the liquid ammonia 19 from the MP ammonia recovery section 5 is deviated and pumped to said intermediate recovery section 32, to provide condensation of the vapour phase 33. The condensate 34 is mixed with the remaining fraction 19B of liquid ammonia from the MP section 5, and directed to the condenser 3.

It should be noted that schemes of Figs. 1 and 2 are simplified block schemes and auxiliary items, as well as compressors, pumps and valves, are not shown.

## Claims

1. A method for revamping a self-stripping urea plant, said plant comprising a high pressure urea synthesis section which includes at least a reactor, a thermal or ammonia stripper and a condenser, a medium pressure treatment section and a low pressure recovery section, the method being **characterized by** the following steps:
- an intermediate recovery section (30) is installed and connected between the high-pressure synthesis section and the medium-pressure section;
- a stream of urea solution (15A), which is delivered by the high pressure section and originally directed to the medium pressure section, is at least partly redirected to said intermediate section (30);
- a flow of concentrated solution (15B) from said intermediate section is directed to said medium pressure section;
- said intermediate section being suitable to decompose the carbamate and recover ammonia contained in said urea solution at an intermediate pressure which is lower than working pressure of said synthesis section and greater than the working pressure of said medium pressure section, obtaining said flow of concentrated solution (15B) at said intermediate pressure.

2. A method according to claim 1, comprising the steps of installing at least a decomposition section (31) and an ammonia recovery section (32) to provide said intermediate section (30), the urea solution (15A) from the high pressure section being at least partly redirected to said decomposition section.

3. A method according to claim 2, comprising the steps of deviating a portion (19A) of a medium pressure liquid stream (19) containing recycled ammonia, obtained in said medium pressure section, to said ammonia recovery section (32) of the intermediate section (30), and directing an ammonia-containing stream delivered by said recovery section to the high pressure condenser.

4. A method according to any of claims 1 to 3, the stream of urea solution which is delivered by the high pressure section being fully redirected to said intermediate section (30).

5. A method according to any of claims 1 to 4, comprising the step of boosting the capacity of the high-pressure synthesis section of the plant.

6. A method according to any of claims 1 to 5, said intermediate section being suitable to operate at a pressure in the range 30 to 100 bar.

7. A self-stripping process for synthesis of urea, where ammonia and carbon dioxide are reacted at a given high pressure in at least one reactor (1) to produce urea; a urea solution (14) produced in said reactor is subject to a thermal or ammonia stripping, a vapour phase (20) containing ammonia and carbon dioxide separated by said stripping is subject to condensation and recycled to said reactor, said stripping and condensation taking place substantially at said high pressure of reaction, and where urea solution is further concentrated and reactants are recovered at a medium pressure and at a low pressure, in respective medium-pressure and low-pressure recovery sections, the process being **characterized in that**:
- at least a part (15A) of a concentrated solution delivered by said thermal or ammonia stripping is subject to a process step of decomposition (31) at an intermediate pressure, said intermediate pressure being lower than said high pressure and greater than said medium pressure, thus obtaining a concentrated solution (15B), and
- said concentrated solution (15B) at intermediate pressure is directed to further treatment at said medium pressure.

8. A process according to claim 7, a vapour phase (33) obtained from said decomposition at intermediate pressure being condensed to obtain a liquid stream containing ammonia (34) at intermediate pressure, said liquid stream being fed to said high-pressure condensation.

9. A process according to claim 7 or 8, the high pressure being greater than 100 bar, the medium pressure being in the range 10 to 20 bar, the low pressure being less than 5 bar, and said intermediate pressure being 30 to 100 bar.

10. A urea plant operating according to the self-stripping process, comprising a high-pressure section including a urea reactor (1), a thermal or ammonia stripper (2) and a condenser (3), and also comprising a medium-pressure urea recovery section (4, 5) and a low-pressure urea recovery section (6, 7), the plant being **characterized by** comprising an intermediate section (30) which is installed and connected between said high-pressure section and said medium-pressure section, said intermediate section comprising at least a decomposition section (31) and an ammonia recovery section (32) operating at an intermediate pressure which is lower than working pressure of said synthesis section and greater than the working pressure of said medium pressure section, and said intermediate section (30) being connected to the high-pressure and medium pressure sections so that the decomposition section (31) of said intermediate section receives at least a part (15A) of a concentrated solution delivered by said thermal or ammonia stripper (2), and a concentrated solution (15B) delivered by said intermediate decomposition section is directed to said medium pressure section.

11. A plant according to claim 10, said ammonia recovery section (32) being connected to receive a portion (19A) of a medium pressure liquid stream (19) containing recycled ammonia, obtained in said medium pressure section, and to deliver an ammonia-containing stream (34) to the condenser (3) of the high pressure section.
